# EUROPEAN PATENT APPLICATION

(11) **EP 1 563 840 A1**
(43) Date of publication of application: **17.08.2005**
(21) Application number: 03758777.1
(22) Date of filing: 22.10.2003
(51) Int. Cl.: A61K 31/7024, A61K 39/395, C07H 15/10

(54) **DENGUE VIRUS INFECTION INHIBITOR**

(30) Priority: 22.10.2002 JP 2002307232
(71) Applicant: Japan Science and Technology Agency, Kawaguchi-shi, Saitama 332-0012 (JP)
(72) Inventor: SUZUKI, Yasuo, Shizuoka-shi, Shizuoka 420-0911 (JP); JWA, Ilpal, Shimizu-shi, Shizuoka 424-0857 (JP)
(74) Representative: Owen, Deborah Jane
(86) International application number: PCT/JP2003/013486
(87) International publication number: WO 2004/037272

(57) **Abstract**

A dengue virus infection inhibitor, as a drug effectively inhibiting the infection with dengue virus, which is **characterized by** containing, as the active ingredient, at least a carbohydrate molecule having as an essential constituent an oligosaccharide chain represented by the following formula (I): Hex¹NAc1-3Hex²-4Hex¹β1- (I) (wherein Hex¹ and Hex² represent a hexose) is provided.

## Description

### Technical Field

The invention of this application relates to a dengue virus infection inhibitor. More particularly, the invention of this application relates to a dengue virus infection inhibitor containing, as the active ingredient, at least a carbohydrate molecule having as an essential constituent an oligosaccharide chain such as a paragloboside saccharide chain.

### Background Art

Dengue fever is an acute infectious disease caused by dengue virus, and is classified based on its clinical characteristics into classical dengue fever (CDF), which has a good prognosis, dengue hemorrhagic syndrome (DHF), which shows the tendency of hemorrhage, and dengue shock syndrome (DSS), which is the most severe form of the disease and is characterized by shock (Yoshihiro Hirabayashi, "Infectious disease syndrome I Ryoikibetsu Shokogun Shirizu No. 23", 1999, pp. 145-149).

CDF is a disease with sudden onset of fever of approximately 40°C, headache, the back and low-back pain, flushed face, conjunctival congestion or the like after a latent period of 3 to 9 days and being accompanied by generalized severe joint pain and muscle pain (Sabin, A.B., American Journal of Tropical Medicine and Hygiene, 1952, Vol. 2, pp. 30-50). In addition, a little bit later, a digestive symptom or upper respiratory inflammation appears. However, these symptoms are self-limiting and cured and recovered in the natural course. On the other hand, DHF or DSS is developed in substantially the same manner as CDF, however, they are different in that the tendency of hemorrhage or shock-like symptoms are prominent in 2 to 6 days, despondency or general debility is marked, and the condition rapidly worsens (Cohen, S.N. et al., Journal of Pediatrics, 1966, Vol. 68, pp. 448-456; Nimmannitya, S. et al., American Journal of Tropical Medicine and Hygiene, 1969, pp. 954-971).

Dengue virus, which is the pathogen of dengue fever, is about 40 to 60 nm in diameter and has an envelope. It has an about 11 kb positive single-stranded RNA, and belongs to the Flaviviridae family together with yellow fever virus, Japanese encephalitis virus and the like in terms of virology. In addition, it is known that dengue virus is classified into 4 serotypes (type 1 to type 4) based on the crossing-over of infection-neutralizing antibodies (Westaway, E.G. et al., Intervirology, 1985, Vol. 24, pp. 183-192; Chambers, T.J. et al., Annual Reviews Microbiology, 1990, Vol. 44, pp. 649-688).

The dengue virus vectors in nature are Aedine mosquitoes. Among them, *Aedes aegypti* mosquitoes which widely inhabit tropical areas become major carrying mosquitoes (Bancroft, T.L. Australasian Medical Gazette, 1906, Vol. 25, pp. 17-18). It is considered that in urban areas, the mosquito-human-mosquito life cycle, in which a human is infected by the bite of a mosquito carrying the virus, to the contrary, a mosquito bites a patient developing viremia thereby acquiring the virus, is established, and in sparsely populated forest areas, the cycle by mosquitoes and monkeys is established (Gubler, D.J., The Arboviruses: Ecology and Epidemiology Vol. 2, 1988, CRC Press, pp. 223-260).

It is known that dengue fever is widely distributed in tropical areas around the world, its infectivity is extremely high, and about 80% of the population is infected during the epidemic. The patients on earth reach 20 million/year (WHO), both epidemic areas and the number of patients have been consistently expanding year by year (Rigau-Perez et al., The Lancet, 1998, Vol. 352, pp. 971-977). In addition, DHF and DSS, which had not been observed before, are frequently developed in various areas recently and the case fatality rate of hemorrhagic fever is 40 % or higher, which is high, therefore, this case is classified as a reemerging infectious disease, and the measures therefor becomes an extremely important issue in public health.

However, the fact is that the information as to a target tissue for infection, a molecule and gene involving the interaction between host and virus in the early stage of infection is very little, and an effective drug for dengue fever or dengue hemorrhagic fever has not yet known. With regard to dengue fever vaccines, the development of attenuated vaccines, inactivated vaccines, subunit vaccines, recombinant vaccines, DNA vaccines and the like has been carried out, however, they have not been put into practical use yet due to the problems of the efficacy and adverse reactions (Chambers, T.J. et al., Vaccine, 1997, Vol. 15, pp. 1494-1502).

On the other hand, by the human genome project and the like, an enormous amount of genetic information has been revealed by genome analysis. A new object in this field is to analyze and elucidate the function of the genes. In order to elucidate the function of the genes, the study on gene expression attracts increasing attention.

For example, it has been reported that ribavirin shows an efficacy against virulent RNA virus infection so far, and ribavirin was expected to work as an effective drug against dengue virus (non-patent document 11). However, it has been reported that ribavirin did not show a preventive effect against dengue virus infection by using rhesus monkeys; to the contrary, it induced anemia or thrombocytosis (Canonico, P.G., Antiviral Research, 1985, Suppl. 1, pp. 75-81). In addition, it has been revealed that, as a receptor for dengue virus, a heparin-like glycosaminoglycan and heparan sulphate, which are present on the cell surface, bind to its outer membrane protein, and a substance inhibiting this binding is expected as a therapeutic drug for dengue virus infection (Marks, R.M. et al., Journal of Medicinal Chemistry, 2001, Vol. 44, No. 13, pp. 2178-2187). However, the host specificity of dengue virus cannot be explained only with this molecule, therefore, it is predicted that another receptor constituent molecule exists.

Further, with regard to dengue fever, many cases in which the infection is promoted by a virus antibody have been reported. The involvement of a complex of an antigen and an antibody, Fc receptor or the like is also suspected, however, the detail mechanism is not known (Schlesinger, J.J. et al., Virology, 1999, Vol. 260, pp. 84-88; Wang, S. et al., Virology, 1995, Vol. 213, pp. 254-257). Many of the studies on the receptor for dengue virus have been carried out at the host individual and cell level, however, it has not been elucidated at the molecular and genetic level yet. It is predicted that a target cell for dengue virus in human is present in a blood system (e.g., Chen, Y.C. et al., Journal of Virology, 1999, Vol. 73, pp. 2650-2657), however, the receptor molecule has not been identified.

Therefore, an object of the invention of this application is to solve the problems as described above and to provide a drug effectively inhibiting the infection with dengue virus.

### Disclosure of the invention

The invention of this application provides, as the one to solve the foregoing problems, firstly, a dengue virus infection inhibitor characterized by containing, as the active ingredient, at least a carbohydrate molecule having as an essential constituent an oligosaccharide chain represented by the following formula (I):

Hex¹NAcβ1-3Hex²β1-4Hex¹β1- (I)

(wherein Hex¹ and Hex² represent a hexose).

The invention of this application provides secondary, a dengue virus infection inhibitor characterized by containing, as the active ingredient, at least a molecule represented by the following formula (II):

(X)ₙ-R (II)

(wherein X represents an oligosaccharide chain represented by the following formula (I):

Hex¹NAcβ1-3Hex²β1-4Hex1β1- (I)

(wherein Hex¹ and Hex² represent a hexose); R is a substrate selected from the group consisting of a hydrogen atom, a substituent having an S, N, O or P atom, a hydrocarbon group, a lipid, a protein and a synthetic polymer, each of which may have a substituent; n is a number of 1 or greater representing the number of the oligosaccharide chains binding to R).

Further, the invention of this application provides thirdly, any one of the foregoing dengue virus infection inhibitors, wherein either a hexose represented by Hex³ or an aminohexose represented by Hex³NAc is beta-1-4 linked to the non-reduced end of the oligosaccharide chain represented by the formula (I).

The invention of this application provides fourthly, any one of the foregoing dengue virus infection inhibitors, wherein Hex¹ in the oligosaccharide chain represented by the formula (I) is glucose (Glc), and Hex² is galactose (Gal) or mannose (Man).

In addition, the invention of this application provides fifthly, a dengue virus infection inhibitor, wherein Hex¹ in the oligosaccharide chain represented by the formula (I) is glucose (Glc), Hex² is galactose (Gal) or mannose (Man), and Hex³ is galactose (Gal) or N-acetyl galactosamine (GalNAc).

Further, the invention of this application provides sixthly, a dengue virus infection inhibitor, wherein the oligosaccharide chain represented by the formula (I) is paragloboside represented by

Galβ1-4GlcNAcβ1-3Galβ1-4Glcβ1- (Ia);

seventhly, a dengue virus infection inhibitor, wherein the oligosaccharide chain represented by the formula (I) is

Galβ1-4GlcNAcβ1-3Manβ1-4Glcβ1- (Ib);

and eighthly, a dengue virus infection inhibitor, wherein the oligosaccharide chain represented by the formula (I) is

GalNAcβ1-4GlcNAcβ1-3Galβ1-4Glcβ1- (Ic).

The invention of this application provides ninthly, a monoclonal antibody to paragloboside represented by the following formula (Ia):

Galβ1-4GlcNAcβ1-3Galβ1-4Glcβ1- (Ia);

tenthly, a monoclonal antibody to an oligosaccharide chain represented by the following formula (Ib):

Galβ1-4GlcNAcβ1-3Manβ1-4Glcβ1- (Ib);

and eleventhly, a monoclonal antibody to an oligosaccharide chain represented by the following formula (Ic):

GalNAcβ1-4GlcNAcβ1-3Galβ1-4Glcβ1- (Ic).

Further, the invention of this application provides twelfthly, a dengue virus infection inhibitor characterized by containing, as the active ingredient, at least any one of the foregoing monoclonal antibodies.

### Brief Description of Drawings

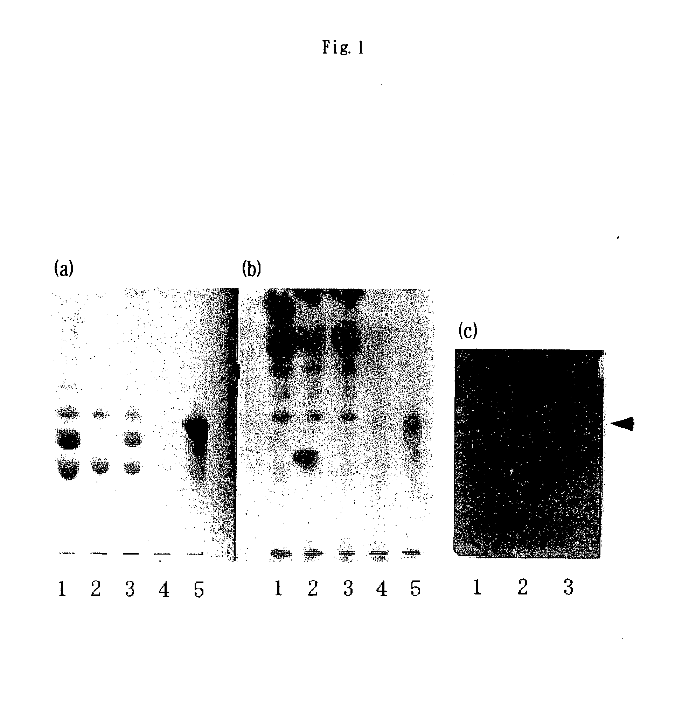

Fig. 1 shows photographs showing the results of TLC analysis and virus binding for the neutral glycolipid fraction of K562 cells after phospholipase C treatment in Examples of the invention of this application (a: sprayed with Dittmer reagent, b: sprayed with orcinol reagent, c: virus-binding assay; 1: neutral glycolipid fraction (untreated), 2: neutral glycolipid fraction (phospholipase C(+)), 3: neutral glycolipid fraction (phospholipase C(-)), 4: phosphatidylcholine (phospholipase C(+)), 5: phosphatidylcholine (phospholipase C(-)); developing solvent: CHCl₃/MeOH/12 mM MgCl₂ (50/40/10, by vol.); used plate: Polygram Sil G).

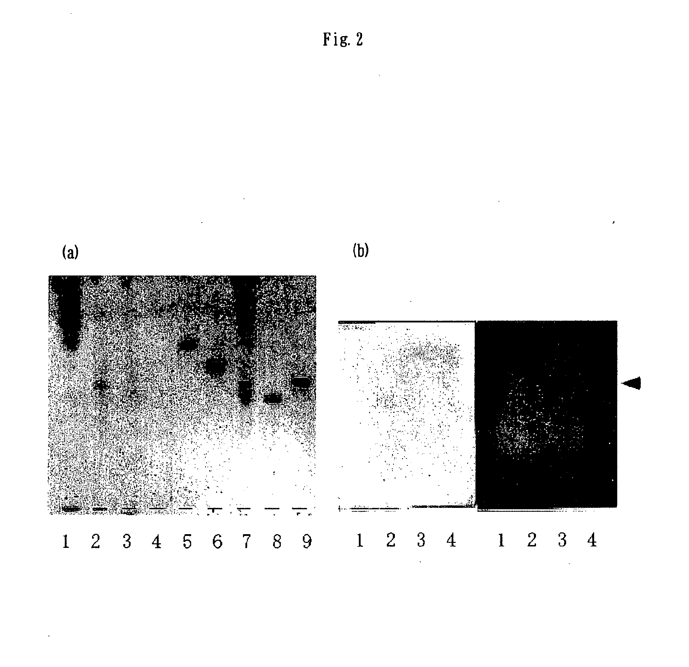

Fig. 2 shows photographs showing the results of virus binding for the neutral glycolipid fractions I to IV of K562 cells in Examples of the invention of this application (a: sprayed with orcinol reagent, b: virus(-), c: virus(+); 1: neutral glycolipid fraction I, 2: neutral glycolipid fraction II, 3: neutral glycolipid fraction III, 4: neutral glycolipid fraction IV, 5: LacCer, 6: CTH, 7: paragloboside, 8: GA₁, 9: globoside; developing solvent: CHCl₃/MeOH/12 mM MgCl₂ (50/40/10, by vol.); used plate: Polygram Sil G).

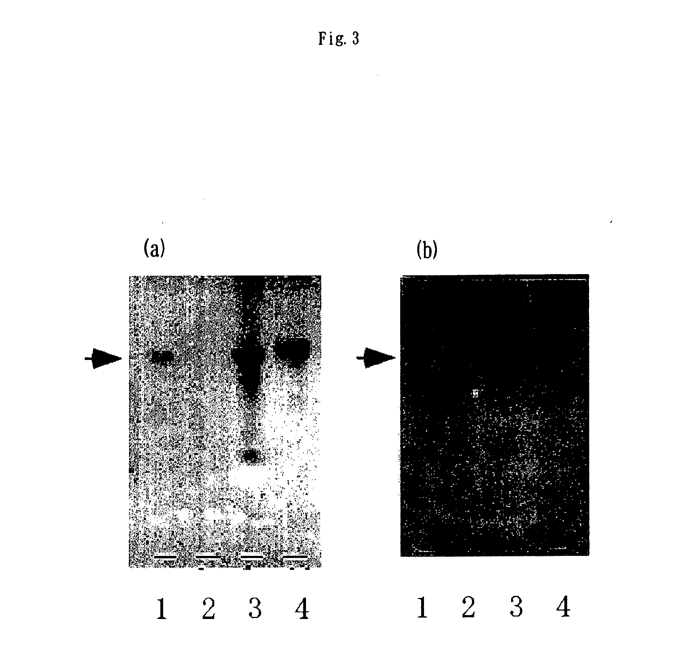

Fig. 3 shows photographs showing the results of virus binding for the neutral glycolipid fraction II-2 of K562 cells in Examples of the invention of this application (a: sprayed with orcinol reagent, b: virus(+); 1: neutral glycolipid fraction II-2, 2: phosphatidylinositol, 3: paragloboside, 4: globoside; developing solvent: CHCl₃/MeOH/12 mM MgCl₂ (50/40/10, by vol.); used plate: Polygram Sil G).

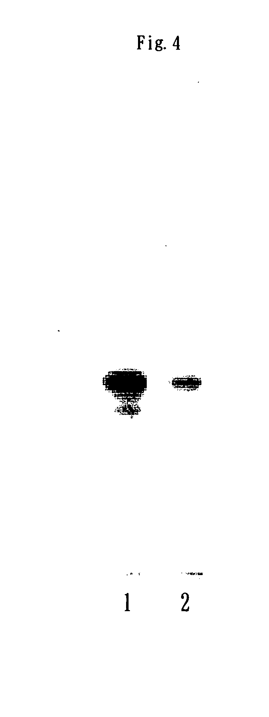

Fig. 4 is a photograph showing the results of immunochemical detection of the purified glycolipid fraction of K562 cells by an anti-PG monoclonal antibody (H11) in Examples of the invention of this application (1: paragloboside, 2: neutral glycolipid fraction II-2; developing solvent: CHCl₃/MeOH/12 mM MgCl₂ (50/40/10, by vol.); used plate: Polygram Sil G).

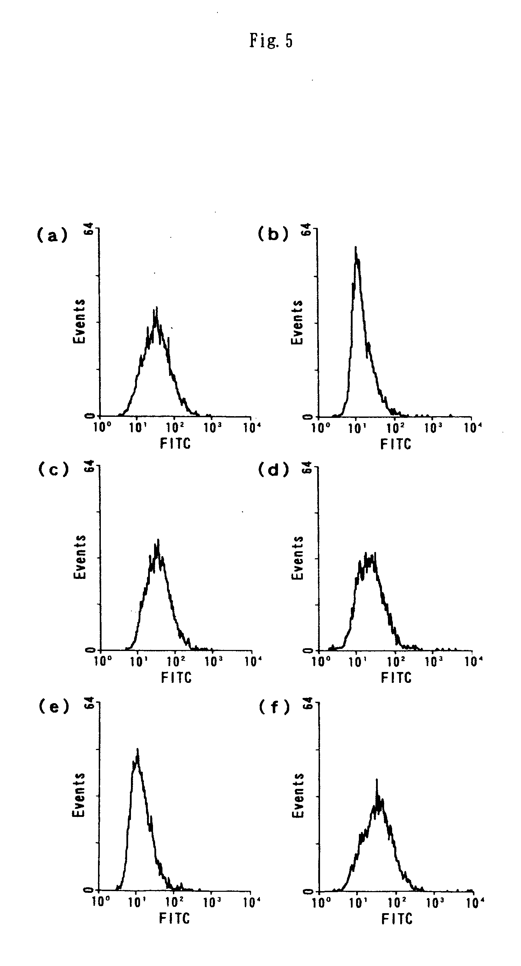

Fig. 5 shows graphs showing the analytical results of an inhibitory effect of paragloboside on binding of dengue virus to K562 cells in Examples of the invention of this application (a: virus(+), b: virus(-), c: paragloboside 100 µM, d: paragloboside 200 µM, e: paragloboside 500 µM, f: GA₁ 500 µM).

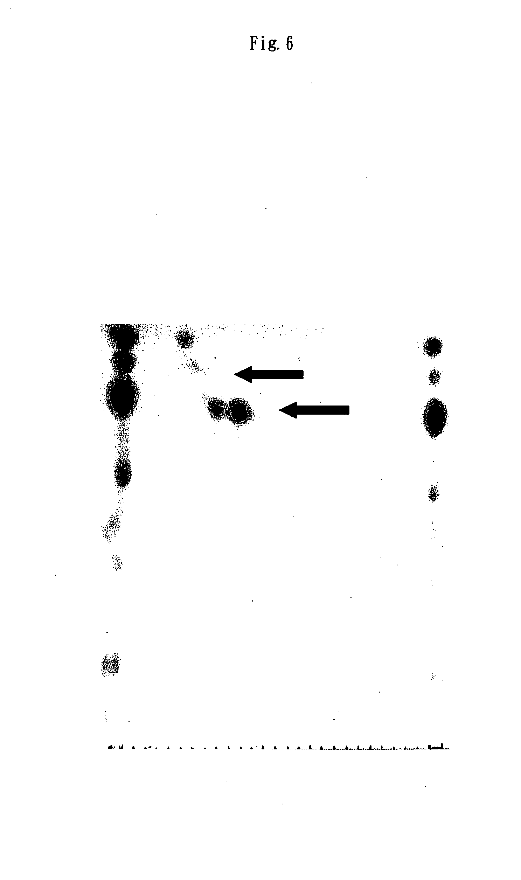

Fig. 6 is a photograph showing the analytical results of an inhibitory effect of paragloboside on binding of dengue virus to K562 cells in Examples of the invention of this application (a: virus(+), b: virus(-), c: paragloboside 100 µM, d: paragloboside 200 µM, e: paragloboside 500 µM, f: GA₁ 500 µM).

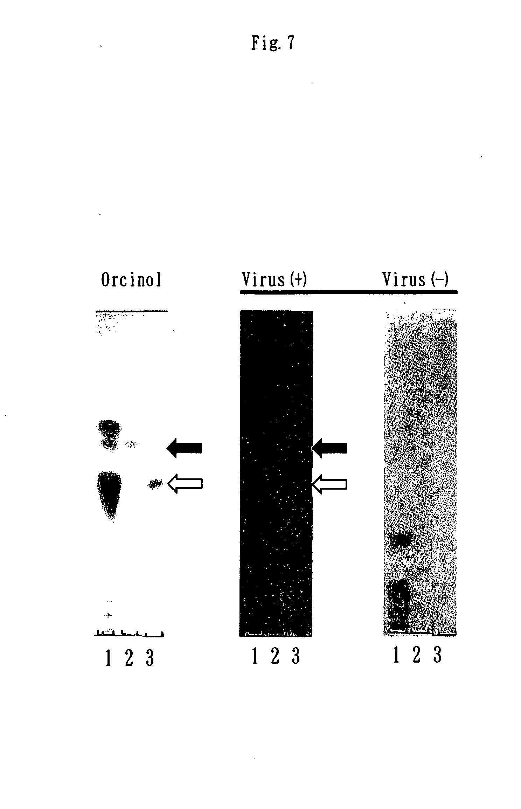

Fig. 7 shows photographs showing the results of analyzing elution patterns by TLC when a dengue virus- binding lipid is isolated from a neutral glycolipid fraction obtained from mosquito-derived C6/36 cells in Examples of the invention of this application.

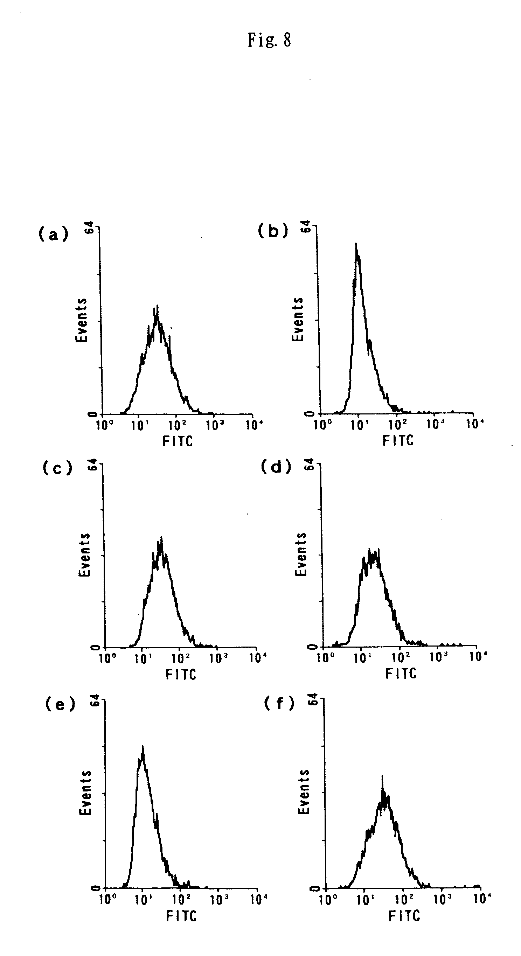

Fig. 8 shows graphs showing the analytical results of binding of dengue virus to a glycolipid obtained from mosquito-derived C6/36 cells in Examples of the invention of this application (1: neutral glycolipid fraction extracted from C6/36 cells, 2: Fractions 13 and 14 purified from 1, 3: Fraction 15 purified from 1).

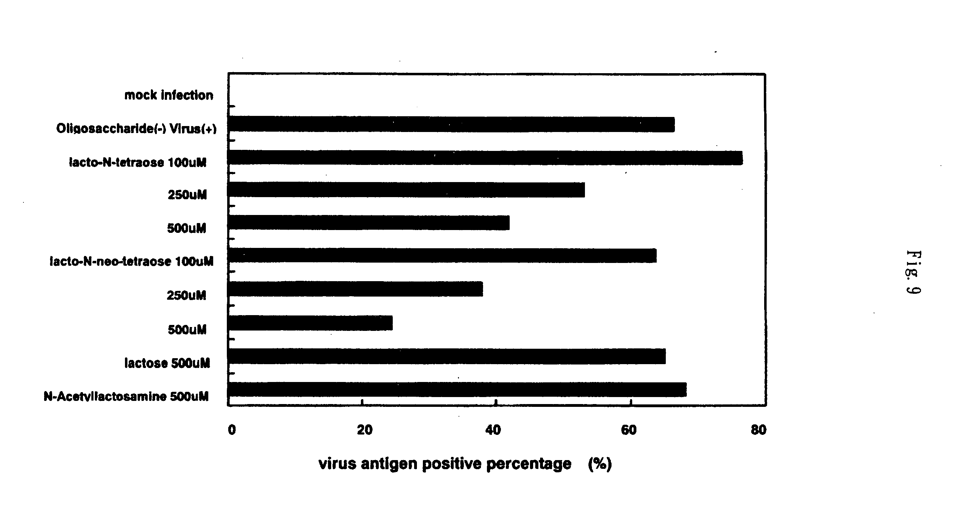

Fig. 9 is a graph showing the results of analyzing inhibition of infection of K562 cell with dengue virus by a paragloboside saccharide chain in Examples of the invention of this application.

### Best Mode for Carrying Out the Invention

The inventors of this application paid attention to the point that the hosts of dengue virus that causes dengue fever are limited to humans and mosquitoes and made intensive studies in order to identify a receptor molecule which these two species share commonly. They investigated, by using culture cells (K562 cells: human myeloid leukemia cell line, and C6/36 cells: derived from *Aedes albopictus)* in which the virus can infect and proliferate, the possibility that the receptor is a lipid, a protein or a sugar-chain adduct thereof by biochemical, immunological and cytobiological techniques. As a result, they succeeded in finding and identifying the receptor for dengue virus, thus the invention of this application has been accomplished.

That is, a dengue virus infection inhibitor of the invention of this application contains, as the active ingredient, a carbohydrate molecule having as an essential constituent an oligosaccharide represented by the following formula (I) :

Hex¹NAcβ1-3Hex²β1-4Hex¹β1- (I).

Such a carbohydrate molecule is not limited to a polysaccharide and may be a glycolipid or a glycoprotein.

In addition, in such a carbohydrate molecule, Hex¹ and Hex² represent a hexose, and specific examples thereof include glucose (Glc), fructose (Fru), galactose (Gal) and mannose (Man). In particular, as Hex¹, Glc is preferred, and as Hex², Gal or Man is preferred.

The dengue virus infection inhibitor of the invention of this application may be the one containing, as the active ingredient, a substance in which the foregoing oligosaccharide region has bound to a synthetic compound or a natural compound such as a hydrocarbon group, a synthetic polymer, a lipid or a protein, in other words, a substance represented by:

(X)ₙ-R (II)

(wherein X represents an oligosaccharide chain represented by the following formula (I):

Hex¹NAcβ1-3Hex²β1-4Hex¹β1- (I)

(wherein Hex¹ and Hex² represent a hexose); R is a substrate selected from the group consisting of a hydrogen atom, a substituent having an S, N, O or P atom, a hydrocarbon group, a lipid, a protein and a synthetic polymer, each of which may have a substituent; n is an integer of 1 or greater representing the number of the oligosaccharides binding to R) as the active ingredient.

At this time, as Hex¹ and Hex², a hexose as described above is exemplified. In addition, preferred examples of R include a hydrogen atom, C₁₋₂₀ acyl groups, which may have a substituent, C₁₋₂₀ alkyl groups, which may have a substituent, substituents such as lactyl groups, amino groups, hydroxyl groups, sulfate groups and phosphate groups, polymer chains such as polyethylene glycol and polyglutamate, dendrimers, polypeptides, lipids such as glycerol and ceramide, serum glycoproteins such as albumin, fetuin and transferrin, DNA and the like. In particular, by using as R a lipid such as glycerol or ceramide, or a serum glycoprotein, which has a low toxicity to humans, the active ingredient of the dengue virus infection inhibitor, which is stable and easy to handle, can be obtained. R may just be bound to the C1 position of βGlc, and may be bound to the C1 carbon of βGlc at any position of R in any form.

The substance represented by the foregoing formula (II) may be the one having a structure in which a plural number (n) of oligosaccharide chains have been bound to one R as described in the reports by, for example, Kitov et al, and Nishikawa et al (Kitov, P.I. et al., Nature 2000, Vol. 403, pp. 669-672; Nishikawa K. et al., Proc. Natl. Acad. Sci., U.S.A. 1999, pp. 7669-7674).

Further, according to the study by the present inventors, it has been revealed that, when either of the hexose represented by Hex³ or the aminohexose represented by Hex³NAc is beta-1-4 linked to the non-reduced end of the oligosaccharide chain represented by the formula (I), the oligosaccharide chain shows a particularly high dengue virus-binding ability, and the effect of the dengue virus infection inhibitor is enhanced. Therefore, the oligosaccharide chain represented by the formula (I) is preferably

Hex³(or Hex³NAc)β1-4Hex¹NAcβ1-3Hex²β1-4Hex1β1- (I').

At this time, as Hex³, glucose (Glc), fructose (Fru), galactose (Gal), mannose (Man) and the like are exemplified. In addition, as Hex³NAc, N-acetylglucosamin (GlcNAc), N-acetylgalactosamin (GalNAc), N-acetylmannosamin (ManNAc) and the like are exemplified. Among them, as Hex³, Gal is preferred, and as Hex³NAc, GalNAc is preferred.

With regard to the dengue virus infection inhibitor as described above, particularly preferred examples of the oligosaccharide chain represented by the formula (I) include paragloboside represented by

Galβ1-4GlcNAcβ1-3Galβ1-4Glcβ1- (Ia),

and oligosaccharide chains represented by

Galβ1-4GlcNAcβ1-3Manβ1-4Glcβ1- (Ib)

and

GalNAcβ1-4GlcNAcβ1-3Galβ1-4Glcβ1- (Ic).

The dengue virus infection inhibitor of the invention of this application is the one utilizing the characteristic that dengue virus specifically recognizes and binds to specific oligosaccharide chains represented by the foregoing formulae, which has been found by the inventors. Therefore, if a monoclonal antibody to these oligosaccharide chains is produced by a known biological method, such a monoclonal antibody also works as the active ingredient of the dengue virus infection inhibitor.

In addition, in the dengue virus infection inhibitor as described above, the composition of components other than the carbohydrate molecule having as an essential constituent an oligosaccharide chain, the substance having an oligosaccharide region or the monoclonal antibody to the oligosaccharide, which is used as the active ingredient, and the form as a pharmaceutical product are not particularly limited. All sorts of components may be contained as long as they do not inhibit the binding of dengue virus to the oligosaccharide, and the dosage form is not particularly limited, either. Specifically, oral administration in the form of a tablet, granule, powder, syrup, or the like, parenteral administration in the form of an injection or the like, and rectal administration in the form of a suppository or the like are exemplified, and the method of administration can be selected depending on the symptoms and conditions of a patient.

In the case where the dengue virus infection inhibitor of the invention of this application is orally administered, it may be in the form of a tablet, lozenge, capsule, arcanum, powder, granule, suspension, emulsion, syrup or the like. In addition, it may be in a slow-release or delayed-release form by formulating it into a coated granule, a multi-layer tablet, a microgranule or the like. In such a form, the dengue virus infection inhibitor may contain a pharmaceutically acceptable binder, sweetener, disintegrator, diluent, artificial flavor, coating agent, preservative, lubricant and/or effect delaying agent, etc. together with the carbohydrate molecule having as an essential constituent an oligosaccharide chain, the substance having an oligosaccharide chain region or the monoclonal antibody to the oligosaccharide chain.

For parenteral administration, the carbohydrate molecule having as an essential constituent an oligosaccharide chain, the substance having an oligosaccharide chain region or the monoclonal antibody to the oligosaccharide chain, which is the active ingredient, may be prepared in a non-toxic and parenterally acceptable diluent or solvent, specifically, in a sterile aqueous solution, oleaginous solution or suspension.

In the dengue virus infection inhibitor of the invention of this application, the carbohydrate molecule having as an essential constituent an oligosaccharide chain, the substance having an oligosaccharide chain region or the monoclonal antibody to the oligosaccharide chain, which is the active ingredient, may be the one that is rectally administered in the form of a suppository or the like. A preferred suppository may be prepared by mixing the active substance with a non-stimulatory excipient that is a solid at ordinary temperatures and melts in the rectum.

The dengue virus infection inhibitor of the invention of this application may be in the form for transdermal administration such as an inhalation spray or ointment, though it is not so common. For example, the inhalation spray may be formulated into a solution, suspension or emulsion and may contain an inhalable propellant with low toxicity such as carbon dioxide or dinitrogen monoxide. On the other hand, preferred examples of the dosage form for transdermal administration include a cream, ointment, gel, jelly, tincture, suspension and emulsion. Such a formulation may contain a pharmaceutically acceptable binder, diluent, disintegrator, preservative, lubricant, dispersant, suspending agent, and/or emulsifying agent.

The dengue virus infection inhibitor of the invention of this application may be produced by a variety of methods that are generally known. For example, it is prepared by grinding, crushing, blending, dispersing, dissolving, suspending, mixing, mingling, combining, emulsifying, or homogenating the carbohydrate molecule having as an essential constituent an oligosaccharide chain, the substance having an oligosaccharide chain region or the monoclonal antibody to the oligosaccharide chain, which is the active ingredient, together with one or more types of suitable carriers, adjuvants, diluents and excipients. In addition, it may be produced by combining one or more of these steps.

In the dengue virus infection inhibitor of the invention of this application, the content of the active ingredient is not particularly limited. For example, the carbohydrate molecule having as an essential constituent an oligosaccharide chain, the molecule having an oligosaccharide chain region or the monoclonal antibody to the oligosaccharide chain, as the active ingredient, can be blended at a concentration of 500 to 1,000 mg/person/day. It is needless to say that the dose to a patient should be determined depending on the symptoms and conditions of the patient based on the diagnosis by the doctor in charge in consideration of the age, sex, body weight, etc. of the patient. Preferably, it is desired to administer it in the range from 10 to 100 mg/kg depending on the body weight of the patient.

The dengue virus infection inhibitor of the invention of this application as described above can be administered to a patient who is suspected to be infected with dengue virus in an early stage of infection for the purpose of suppressing the onset of the symptoms of dengue fever. In addition, if the dengue virus infection inhibitor of the invention of this application is administered to a patient showing the symptoms of dengue fever, it will become possible to treat dengue fever. Further, the dengue virus infection inhibitor of the invention of this application effectively works as a preventive agent for infection with dengue virus by administering it to inhabitants in the epidemic areas of dengue fever or travelers to the epidemic areas.

Hereunder, the invention of this application will be described in more detail with reference to Examples. It is needless to say that the invention of this application is not limited to the following Examples.

### Examples

### <Example 1> Isolation and purification of dengue virus- binding lipid from K562 cells (human myeloid leukemia cell line)

### (1) Isolation and purification of neutral glycolipid

To K562 cells (in 26 pieces of T-225 flasks), 0.5 ml of chilled PBS(-) was added, suspended and centrifuged at 4°C. To the obtained pellet, 5 ml of purified water was added and well suspended. Then, 100 ml of CHCl₃/MeOH (1/1, by vol.) was added, and extraction of the total lipid was carried out for 1 hour while stirring. The extract was filtered, and the same treatment was carried out again for the residue. The obtained filtrates were combined and transferred to a round-bottomed flask, and the solvent was removed. To the dried and solidified total lipid fraction, 30 ml of MeOH was added, and sonication was carried out for 5 minutes. Then, 300 µl of 10 N NaOH was added and incubated overnight at 37°C.

After the reaction, 3 ml of 1 N AcOH was added and well suspended. Then, sonication was carried out for 5 minutes and the solvent was removed again. To the dried and solidified total lipid fraction, 5 ml of purified water was added and well suspended, and sonication was carried out for 5 minutes. The same treatment was carried out again, and the combined suspension was dialyzed overnight at 4°C. Lyophilization was carried out, and the obtained product was used as a total lipid fraction.

The obtained total lipid fraction was dissolved in 5 ml of CHCl₃MeOH/H₂O (30/60/8, by vol.) and well suspended, then sonication was carried out for 5 minutes. The same treatment was carried out again, and the combined suspension was applied to a DEAE-Sephadex A-25 (acetate form, gel volume: 100 ml) column, which had been equilibrated with the same solvent in advance. CHCl₃/MeOH/H₂O (30/60/8, by vol.) was passed through in a volume of 10 times the gel volume, and the fraction was collected, which was used as a neutral glycolipid fraction, then the solvent was removed.

To the neutral glycolipid fraction, 2 ml of CHCl₃/MeOH (1/1, by vol.) was added and well suspended.

### (a) TLC analysis of neutral glycolipid fraction

The obtained neutral glycolipid fraction (5 µl), phosphatidylethanolamine (PE) (1 µl), LacCer (Galβ1-4Glcβ1-1 'Cer) (1 µl), Ceramide monohexioside (CMH: Glcβ1-1'Cer) (2 µl), Ceramide trihexoside (CTH: Ga!al-3Ga!pl-4G!cpl-l'Cer) (2 µl), phosphatidylcholine (PC) (2 µl), phosphatidylserine (PS) (2 µl) and phosphatidylinositol (PI, derived from bovine liver, Sigma Co.) (2 µl) were spotted on HPTLC plates, developed with acetone once, air dried, and developed with CHCl₃/MeOH/12 mM MgCl₂ (60/35/8, by vol). On one plate, orcinol reagent was sprayed and the plate was heated at 110°C, and on one plate, Dittmer reagent was sprayed and color reaction was developed.

### (b) Alkaline hydrolysis of neutral glycolipid fraction again

The solvent of the neutral glycolipid fraction was removed, and 5 ml of MeOH was added and well suspended. Then, sonication was carried out for 5 minutes. Thereto was added 50 µl of 10 N NaOH, and incubated at 37°C for 3 hours. After the reaction, 5 ml of 0.1 N AcOH was added and well suspended. Then, sonication was carried out for 5 minutes and the solvent was removed again. Thereto was added 5 ml of purified water and well suspended, and sonication was carried out for 5 minutes. The same treatment was carried out again, and the combined suspension was dialyzed overnight at 4°C. Then lyophilization was carried out.

### (c) Phospholipase C treatment

It was considered that phospholipid was contained in the neutral glycolipid fraction; therefore, phospholipase C treatment was carried out under the condition that PC was completely hydrolyzed.

First, the neutral glycolipid fraction (50 µl) or PC (5 µl), as a positive control, was added to 2 test tubes, respectively, and evaporated and solidified under a nitrogen gas stream. Then, 100 µl of ether/ethanol (98/2, by vol.) was added to the respective tubes, and sonication was carried out for 1 minute. To one of the test tubes for each sample, 100 µl of a solution of phospholipase C (derived from Clostridium perfingens, SIGMA Co.) (0.1 mg/100 µl) dissolved in 0.1 M Tris buffer (Tris (hydroxylmethyl) aminomethane) (pH 7.2) containing CaCl₂ was added, and to the other remaining test tube for each sample, 100 µl of only Tris buffer (pH 7.2) containing CaCl₂ was added. Then, the mixture was well suspended, and reacted for 3 hours in a water bath at 25°C.

After the reaction, the solvent was removed under a nitrogen gas stream, and the product was desalted with a reverse-phase column. The desalted fraction was evaporated and solidified under a nitrogen gas stream, and CHCl₃/MeOH (1/1. by vol.) was added to the neutral glycolipid fraction in an amount of 30 µl, and to PC in an amount of 15 µl, and well suspended. Then, the solutions were spotted on TLC plates (Polygram Sil G). Then, the TLC plates were developed with acetone once, air dried, and developed with CHCl₃/MeOH/12 mM MgCl₂ (50/40/10, by vol). On one plate, orcinol reagent was sprayed and the plate was heated at 110°C, and on one plate, Dittmer reagent was sprayed and color reaction was developed.

In addition, virus-binding assay was carried out.

The results are shown in Fig. 1. The change in virus binding by phospholipase C treatment was not observed. After the phopholipase C treatment, several bands of phospholipids other than PC were observed. Therefore, the neutral glycolipid fraction was further purified into 4 fractions using Iatrobeads column.

The total amount of the neutral glycolipid fraction was treated with phopholipase C, and the total amount of the reaction solution was dialyzed overnight at 4°C, whereby it was desalted. To the dried specimen, 1 ml of CHCl₃/MeOH (8/2, by vol.) was added, suspended, and sonication was carried out for 5 minutes. This solution was applied to Iatrobeads column, and elution was continuously carried out with 4 types of solvents (1) CHCl₃/MeOH (8/2, by vol.), (2) CHCl₃/MeOH/H₂O (65/25/4, by vol.), (3) CHCl₃/MeOH/H₂O (60/35/8, by vol.) and (4) CHCl₃/MeOH/H₂O (50/40/10, by vol.), whereby the neutral glycolipid fraction was further separated into 4 fractions (referred to as neutral glycolipid fractions I to IV, respectively).

These were evaporated and solidified under a nitrogen gas stream, and 1 ml of CHCl₃/MeOH (1/1, by vol.) was added, respectively. Then, 5 µl thereof for each fraction was spotted on TLC plates (Polygram Sil G). The plate was developed with acetone once again, and developed with CHCl₃/MeOH/12 mM MgCl₂ (50/40/10, by vol). Then, orcinol reagent or Dittmer reagent was sprayed and color reaction was developed.

### (d) Chemical analysis and investigation of virus binding for neutral glycolipid fractions I to IV

The neutral glycolipid fractions I to IV obtained in the foregoing (c) (10 µl each), and as a reference standard, LacCer (1 µl), CTH (2 µl), paragloboside (PG: Galβ1-4GlcNAcβ1-3Galβ1-4Glcpβ1-1'Cer) (2 µl), GA₁ (Galβ1-3GalNAβ1-4Galcβ1-4Glcβ1-1'Cer) (2 µl), globoside (GaINAcβ1-3Galα1-4Galβ1-4Glcβ1-1'Cer) (2 µl) were spotted on TLC plates (Polygram Sil G), developed with acetone once, and developed with CHCl₃/MeOH/12 mM MgCl₂ (50/40/10, by vol). Then, orcinol reagent was sprayed and the plate was heated at 110°C, whereby color reaction was developed. With regard to the neutral glycolipid fractions I to IV, TLC/virus-binding assay was carried out by the method described above.

The results are shown in Fig. 2.

From Fig. 2, it was suggested that, among the neutral glycolipid fractions I to IV, the neutral glycolipid fraction II shows strong binding to the virus and has a very close molecular structure to the neutral glycolipids called CTH, PG and globoside.

### (e) Scraping and extraction of target glycolipid from TLC plate

Subsequently, the region showing binding to virus was scraped from the TLC plate, and extraction was carried out, whereby a neutral glycolipid fraction containing a target substance was obtained.

The TLC plate (Polygram Sil G) was developed with CHCl₃/MeOH/H₂O (50/40/10, by vol.) and air dried in advance as a pretreatment. The neutral glycolipid fraction II was evaporated and solidified under a nitrogen gas stream, and 0.1 ml of CHCl₃/MeOH (1/1, by vol.) was added thereto. Then, the total amount thereof was spotted on the plate to be 1 µl/mm lane. Then, the plate was developed with acetone once, and developed with CHCl₃/MeOH/12 mM MgCl₂ (50/40/10, by vol).

Both ends of the TLC plate were cut off, and orcinol reagent was sprayed. Then, the plate was heated at 110°C, to develop color reaction, and the band of the target glycolipid was confirmed.

The remaining region corresponding to the band on the plate was cut off with a pair of scissors, and the silica gel was scraped from the cut-off TLC plate. Thereto was added 25 ml of CHCl₃/MeOH/H₂O (30/60/8, by vol.), stirred for 1 hour and extraction was carried out. The extract was filtered through a cotton plug, and the solvent was removed from the filtrate. Thereto was added 1 ml of CHCl₃/MeOH (8/2, by vol.), and sonication was carried out. Then, the mixture was applied to Iatrobeads column (gel volume: 1 ml), prepared with a Pastetir pipette.

Fifteen milliliters of CHCl₃/MeOH (8/2, by vol.) were passed through the column, and the fraction was collected (neutral glycolipid fraction II**-**1)**.**

Subsequently, the elution solvent was changed to CHCl₃/MeOH/H₂O (30/60/8, by vol.), and 20 ml of the solvent was passed through the column (neutral glycolipid fraction II-2).

The solvent was removed from each of the fractions, and 0.5 ml of CHCl₃/MeOH (1/1, by vol.) was added. Five microliters of each of the solutions were spotted on a TLC plate, developed with acetone once, and developed with CHCl₃/MeOH/12 mM MgCl₂ (50/40/10, by vol). Then, orcinol reagent was sprayed, and the plate was heated at 110°C, to develop color reaction. At this time, as a reference standard, PG, globoside and CTH were also spotted on a TLC plate in 2 µl aliquots, respectively.

### (2) Analysis of virus binding to neutral glycolipid fraction

The neutral glycolipid fraction II-2 (10 µl) obtained by the foregoing method described in (1), PI (10 µl), PG (2 µl) and globoside (1 µl) were spotted on a TLC plate (Polygram Sil G), developed with acetone once, and developed with CHCl₃/MeOH/12 mM MgCl₂ (50/40/10, by vol). Then, orcinol reagent was sprayed, and the plate was heated at 110°C, to develop color reaction. TLC/virus-binding assay was carried out by the foregoing method.

The results are shown in Fig. 3.

From Fig. 3, it was confirmed that in the neutral glycolipid fraction II-2, a glycolipid migrating at the position very close to that of PG on the TLC plate is included and further, that this glycolipid shows strong binding to dengue virus. In addition, also for PG, which is a reference standard, a similar virus binding was observed.

### (3) Structural analysis of binding lipid contained in neutral glycolipid fraction

From the above, it was suggested that a saccharide chain molecule showing binding to dengue virus has a saccharide chain structure very close to that of PG. Therefore, immunochemical analysis was carried out by using an anti-PG monoclonal antibody.

### (a) Immunochemical analysis

The neutral glycolipid fraction II-2 (10 µl) and PG (2 µl) were spotted on a TLC plate (Polygram Sil G), and continuously developed with acetone and CHCl₃/MeOH/12 mM MgCl₂ (50/40/10, by vol). Blocking of the TLC plate was carried out overnight at 4°C in a solution of 1% BSA-PBS (bovine serum albumin-phosphate buffered saline). After the plate was reacted with an anti-PG monoclonal antibody (clone H11) (5 ml) diluted to 5000-fold with PBS containing 1% BSA for 1 hour, washed with PBS (10 ml) containing 0.05 % Tween 20 for 3 minutes, and this washing was repeated 5 times. After the washing, the plate was reacted with an HRP (horseradish peroxidase)-labeled goat anti-mouse IgG + IgM (5 ml) (American Qualex Antibodies) diluted to 5000-fold with PBS containing 1% BSA for 1 hour. The plate was washed with PBS(-) for 3 minutes, and this washing was repeated 5 times. Finally, the plate was reacted with a color-developing substrate solution [0.1 M citrate buffer pH 6.0 (5 ml), 0.11 M 4-chloro-1-naphthol in CH₃CN (100 µl), 0.06 M DEPDA in CH₃CN (100 µl), 30 % H₂O₂ (10 µl)], and spots were detected.

The results are shown in Fig. 4.

From the Fig. 4, it was found that in the neutral glycolipid fraction II-2, the same glycolipid as in PG is contained.

From the above, it was confirmed that the dengue virus-binding lipid in K562 cells is a neutral glycolipid having a paragloboside (PG) saccharide chain structure represented by the following formula:

Galβ1-4GlcNAcβ1-3Galβ1-4Glcβ1-1Ceramide

(wherein Gal represents galactose, GlcNAc represents N-acetylglucosamine, and Glc represents glucose).

### <Example 2> Analysis of inhibitory effect of PG on binding of dengue virus to K562 cells surface using flow cytometer

After K562 cells were cultured until confluent, the cells were dispensed into a microtube to be 2 x 10⁵ cells/0.1 ml. After this microtube was centrifuged at 4°C for 2 minutes at 10,000 rpm, the obtained cell pellet was washed once with 1 ml of PBS containing 0.1 % BSA. After a dengue virus solution (2,000 units/ml) and PG (0, 100, 200 or 500 µM) or GA₁ (500 µM), which is a reference glycolipid, were preincubated in PBS containing 0.1 % BSA at 4°C for 1 hour, 100 µl of each of the solutions was added to K562 cells, and the virus was allowed to bind to the cell at 4°C for 1 hour. Incidentally, as virus(-), only PBS containing 0.1 % BSA was added in the same amount. After the reaction, the cells were washed 3 times with 1 ml of chilled PBS(-), 100 µl of a solution of human anti-flavivirus antiserum (lot No. 1), which had been diluted to 200-fold, was added, respectively, as a primary antibody, well suspended, and reacted at 4°C for 30 minutes. The cells were washed 3 times with 1 ml of chilled PBS(-), and 100 µl of a solution of a FITC-labeled goat anti-human IgG (Zymed), which had been diluted to 50-fold, was added, respectively, as a secondary antibody and shielded from light. Then, they were reacted at 4°C for 30 minutes. After the reaction, the cells were washed 3 times with 1 ml of chilled PBS(-), and to the obtained pellet, 0.5 ml of chilled PBS(-) was added and well suspended. Then, measurement was carried out with a flow cytometer (Epics, U.S.A).

Inhibition of binding of dengue virus to K562 cells by PG was investigated, and the PG concentration dependency is shown in Fig. 5.

From Fig. 5, it was demonstrated that the binding of dengue virus to K562 cells are inhibited depending on the concentration of PG. The reference glycolipid, GA₁, had a very similar physical property to that of PG in that it is a neutral tetrasaccharide and that it has a galactose residue at the non-reduced end in the molecule. However, GA₁ could not inhibit the binding of dengue virus to K562 cell even at a concentration of 500 µM.

From above, it was demonstrated that PG specifically binds to dengue virus and the attachment of the virus to the surface of a host cell is inhibited by this specific binding.

### <Example 3> Investigation of inhibitory activity of paragloboside on binding of dengue virus to K562 cell surface

### (1) Concentration of dengue virus

After K562 cells were cultured until confluent, the cells were dispensed into a microtube to be 4 x 10⁵ cells/0.1 ml. After this microtube was centrifuged at 4°C for 3 minutes at 3,500 rpm, the obtained cell pellet was washed once with 1 ml of PBS containing 0.1% BSA. After it was further centrifuged at 4°C for 3 minutes at 3,500 rpm, a dengue virus stock solution was diluted with PBS containing 0.1% BSA to the concentrations of 5,000 units/ml, 2,000 units/ml, 1,000 units/ml, and 500 units/ml, respectively. Then, 100 µl of each of the solutions was added to the K562 cells, and reacted at 4°C for 1 hour.

Incidentally, as virus(-), a solution to which only PBS containing 0.1 % BSA was added in the same amount was used.

After the reaction, the cells were washed 3 times with 1 ml of chilled PBS(-), 100 µl of a solution of human anti-flavivirus antiserum (lot No. 1), which had been diluted to 200-fold, was added, respectively, as a primary antibody, well suspended, and reacted at 4°C for 30 minutes. The cells were washed 3 times with 1 ml of chilled PBS(-), and 100 µl of a solution of a FITC standard goat anti-human IgG (Zymed), which had been diluted to 50-fold, was added, respectively, as a secondary antibody and shielded from light. Then, they were reacted at 4°C for 30 minutes.

After the reaction, the cells were washed 3 times with 1 ml of chilled PBS(-), and to the obtained pellet, 0.5 ml of chilled PBS(-) was added and well suspended. Then, measurement was carried out with a flow cytometer (Epics, U.S.A).

Hereinafter, the virus concentration was set at 2,000 units/ml.

Inhibition of the binding of dengue virus to K562 cells by PG was investigated, and the PG concentration dependency is shown in Fig. 6.

### (2) Investigation of dilution concentration of primary antibody

Based on (1), the concentration of dengue virus was set at 1,000 units/ml, and a binding assay was carried out. Here, a primary antibody was diluted to 200-fold, 400-fold, 800-fold and 1,600-fold with chilled PBS(-). Incidentally, as virus(-), a primary antibody diluted to 200-fold was used. The experimental method was in accordance with (1).

Hereinafter, as the primary antibody, the one diluted to 200-fold was used.

### (3) Effect of paragloboside on virus binding

K562 cells (1 x 10⁵ cells) were seeded on a 24-well culture plate, and the total volume was made up to 1 ml with RPMI medium. Paragloboside was added so as to give a final concentration of 0 µM, 100 µM, 200 µM or 500µM by diluting it, and reacted at 37°C for 1 hour. This solution was centrifuged at a low speed, and 1 ml of chilled PBS containing 0.1% BSA was added to the obtained pellet. The solution was transferred to a microtube, and virus binding was evaluated by the method described in (1).

From Fig. 6, it was clearly observed that, when dengue virus and paragloboside were incubated in advance, inhibition of the binding of dengue virus to K562 cells is caused depending on the concentration of paragloboside.

In addition, a similar experiment was carried out by using, as a negative control, GA1 (asialo-GM1) (Galβ1-3GalNAcβ1-4Galβ1-4Glcβ1-1Cer), which is a structurally-similar neutral glycolipid having a saccharide chain of tetrasaccharide structure, and as a result, the binding of dengue virus to the cell surface was not affected at all.

Accordingly, it was confirmed that inhibition of dengue virus binding is a characteristic specific to the structure of a paragloboside saccharide chain.

From the above, it was demonstrated that paragloboside present on the surface of K562 cell is a receptor for dengue virus itself, or functions as a part thereof.

### <Example 4> Isolation and purification of dengue virus- binding lipid from culture cell line derived from mosquito and identification thereof

### (1) Culture of C6/36 cell

As a target cell for dengue virus, C6/36 cell line derived from *Aedes albopictus* showing sensitivity to this virus and having a further higher virus-producing ability was used. C6/36 cells were cultured at 28°C in MEM medium containing 10 % inactivated fetal bovine serum and 1 % nonessential amino acids.

The subculture of the cell line was carried out by adding fresh MEM medium to one-tenth of the previous culture solution to make the total volume up to 20 ml and seeding it in a T-75 flask.

### (2) Extraction of total lipid

C6/36 cells were harvested from 15 L of the culture solution. To the obtained cell pellet, 1 L of CHCl₃MeOH/H₂O (10/10/1, by vol.) was added and well suspended, and extraction was carried out for 24 hours.

This solution was filtrated, and the same treatment was carried out again for the residue. The obtained supernatants were combined and transferred to a round-bottomed flask, and the solvent was removed. Further, 100 ml of MeOH was added and well suspended, and sonication was carried out for 10 minutes. Then, 1 ml of 10 N NaOH was added and incubated at 37°C for 15 hours.

After the reaction, 1 ml of 10 N AcOH was added and well suspended. Then, sonication was carried out for 5 minutes and the solvent was removed again. Thereto was added 20 ml of purified water and well suspended, and sonication was carried out for 30 minutes. The same treatment was carried out again, and the combined suspension was dialyzed at 4°C for 46 hours. Then, lyophilization was carried out, and the obtained product was used as a total lipid fraction.

### (3) Ion exchange column chromatography

To the obtained total lipid fraction, 100 ml of CHCl₃/MeOH/H₂O (30/60/8, by vol.) was added and well suspended, and sonication was carried out for 10 minutes. This suspension was applied to a DEAE-Sephadex A-25 (acetate form, gel volume: 400 ml) column, which had been equilibrated with the same solvent in advance. CHCl₃/MeOH/H₂O (30/60/8, by vol.) was passed through in a volume of 47.5 times the gel volume, and the fraction was collected, which was used as a neutral glycolipid fraction.

### (4) Purification of neutral glycolipid fraction

The neutral glycolipid fraction was dissolved in 3 ml of CHCl₃/MeOH (8/2, by vol). One milliliter of this fraction was injected into an HPLC system (PU-2080 Plus, Nihon Bunko Co.) provided with a Senshu Pak AQUASIL-SS-3251 column (8 φ x 250 mm), which had been equilibrated with the same solvent in advance. By passing through 100 ml in total of a linear concentration gradient from the same solvent (100%) to (the same solvent) : CHCl₃/MeOH/H₂O (60/35/8, by vol.) (30/70, by vol.), the column eluate was collected in 2 ml aliquots. The respective fractions were spotted on an HPTLC plate in 2 µl aliquots, respectively, and the plate was developed with CHCl₃/MeOH/12 mM MgCl₂ (60/35/8, by vol). Then, orcinol reagent was sprayed, and the plate was heated at 110°C, to develop color reaction, whereby elution of the glycolipid was confirmed.

### (5) Reactivity of neutral glycolipid fraction with dengue virus

On 3 TLC plates (Polygram Sil G, Marchery Nagel), the neutral glycolipid fraction was spotted in 1 µl aliquots and air dried. Then, the plates were developed with nPrOH/H₂O/NH₄OH (75/25/5, by vol.) and air dried. Then, on one of the plates, orcinol reagent was sprayed, and the plate was heated at 110°C, to develop color reaction. With regard to the remaining 2 plates, PBS was added and the plates were impregnated with PBS slowly and sucked. Then, blocking was carried out with Solution A (PBS containing 1 % ovalbumin and 1% polyvinylpyrrolidone) at room temperature for 1 hour.

After the reaction, to virus(+), 3 ml of dengue virus (D2Th7 strain) suspension prepared at 1,000 units/ml with Solution A was added, and to virus(-), only Solution A was added in the same amount, and let stand overnight at 4°C. The plates were washed 3 times for 3 minutes with PBS(-).

Subsequently, 2.5 ml of human anti-flavivirus antiserum diluted to 1,000-fold with Solution A was added and reacted at room temperature for 1 hour. The plates were washed 3 times for 3 minutes with PBS(-). Finally, the plates were reacted with 2.5 ml of a color-developing substrate solution (0.1 M citrate buffer pH 6.0 (2.5 ml), 0.11 M 4-chloro-1-naphthol in CH₃CN (50 µl), 0.06 M DEPDA in CH₃CN (50 µl), 30% H₂O₂ (5 µl)), and bound virus particles were detected. Incidentally, dengue virus (D2Th7 strain) and human anti-flavivirus antiserum were provided by Professor Akira Igarashi and Professor Koichi Morita of Institute of Tropical Medicine, Nagasaki University.

### (6) TLC analysis of purified glycolipid

The isolated neutral glycolipid was spotted on an HPTLC plate in 1.5 to 2 µl aliquots. The plate was developed with nPrOH/H₂O/NH₄OH (75/25/5, by vol.) and orcinol reagent was sprayed. Then, the plate was heated at 110°C, and color reaction was developed.

In addition, each fraction was spotted on 3 TLC plates (Polygram Sil G) in 0.5 to 1 µl aliquots, developed with acetone once and air dried. Then, the plate was developed with nPrOH/H₂O/NH₄OH (75/25/5, by vol.) and air dried. On one of the plates, orcinol reagent was sprayed, and the plate was heated at 110°C, to develop color reaction. With regard to the remaining 2 plates, virus-binding assay was carried out by the method described in (5).

Subsequently, search for dengue virus-binding lipid from mosquito-derived C6/36 cells, which is a cell line with a high sensitivity to dengue virus was carried out.

A dengue virus-binding lipid was eluted in the neutral glycolipid fraction due to fractionation by anion exchange column chromatography in the same manner as the case of K562 cells.

In order to isolate a binding lipid from this fraction, HPLC provided with a silica column was carried out. The results of analyzing the elution patterns by TLC are shown in Fig. 7. The 2 types of glycolipids indicated by the arrows in the figure had a binding ability. Under the conditions of HPLC performed this time, these 2 types of glycolipids were completely isolated.

Therefore, the results of analyzing the binding of the purified glycolipids to dengue virus are shown in Fig. 8. It was demonstrated that the purified glycolipids contained in Fractions 13, 14 and 15 binds to the virus very strongly. From the structural comparison with a standard glycolipid derived from an insect, the structure of the glycolipid contained in Fractions 13 and 14 is GlcNAcβ1-3Manβ1-4Glcβ1-1Cer (wherein Man represents mannose and Cer represents ceramide), on the other hand, the structure of the glycolipid contained in Fraction 15 is totally new, and was deduced to be Galβ1-4GlcN Acβ1-3Manβ1-4Glcβ1-1Cer.

The structures of paragloboside isolated from K562 cell as a binding lipid and glycolipids isolated from the foregoing Examples are summarized as follows:
Paragloboside: Galβ1-4GlcNAcβ1-3Galβ1-4Glcβ1-1Cer
Fraction 15: Galβ1-4GlcNAcβ1-3Manβ1-4Glcβ1-1Cer
Fractions 13 and 14: GlcNAcβ1-3Manβ1-4Glcβ1-1Cer.

From these structures, it was suggested that a minimum glycolipid structure necessary for binding to dengue virus is GlcNAeβ1-3Man (or Gal) β1-4Glcβ1-, and moreover, that its binding is enhanced by connecting Gal to the non-reduced end through a β1-4 linkage.

### <Example 5> Investigation of inhibitory activity of paragloboside saccharide chain on infection of K562 cells with dengue virus

Any of the paragloboside saccharide chains used in the foregoing Examples was a substance binding to ceramide.

Therefore, in order to confirm that the inhibitory effect of the paragloboside saccharide chain on dengue virus binding does not depend on the bound substrate for paragloboside and a similar effect can be obtained even with a bound substrate other than ceramide, the virus binding inhibitory effect was studied for only the paragloboside saccharide chain without a substrate at the end.

### (1) Preparation of paragloboside saccharide chain molecule and other oligosaccharides

The following oligosaccharides were used. Paragloboside saccharide chain: Gatβ1-4GlcNAcβ1-3Galβ1-4Glc
Lacto-N-tetraose: Galβ1-3GlcNAcβ1-3Galβ1-4Glc
Lactose: Galβ1-4Glc
N-acetyllactosamine (LacNAc): Galβ1-3GlcNAc

In any cases, 1 mM stock solution was prepared with sterile PBS(-). This solution was appropriately diluted and used for the infection inhibition test.

### (2) Preparation of dengue virus solution

Dilution was carried out with serum-free RPMI medium (SF-RPMI) containing 0.2% BSA so as to give a final virus concentration of 3 x 105 FFU (focus forming unit)/sample, and the solution was stirred well and placed on ice.

### (3) Pretreatment of dengue virus with oligosaccharide

To 630 µl (virus volume per 1 sample) of the dengue virus solution prepared in (2) described above, an equivalent amount of a diluted solution containing an oligosaccharide was added, stirred well, and incubated at 28°C for 30 minutes. This solution was used as a pretreated virus solution.

In addition, as a negative control, the one obtained by mixing PBS(-) with the virus solution was used.

### (4) Virus infection test

After K562 cells were washed once with SF-RPMI, the cells were prepared at 1 x 10⁶ cells/ml with 0.1 % BSA-SF-RPMI. This cell suspension was dispensed in 100 µl aliquots into tubes. Thereto was added the pretreated virus solution, stirred gently, and infection of cells was carried out at 37°C for 2 hours. During this time, the solution in the tube was gently stirred every 20 minutes. After the solution was centrifuged at 3,400 rpm for 3 minutes, the virus solution was removed, and the cells were washed 3 times with 0.1 % BSA-SF-RPMI. To the cell pellet, 2 ml of RPMI medium containing 2% serum (2% FCS-RPMI) was added, and suspended. Then, the suspension was transferred to a 35-mm dish and cultured at 37°C.

After being cultured for a predetermined period of time, the cells were collected, and detection of a virus antigen was carried out.

### (5) Detection of virus antigen (determination of virus infectivity titer)

The collected cells were transferred to a new tube and washed once with PBS(-). To the cell pellet, 200 to 600 µl of 4% paraformaldehyde/PBS (fixation solution) was added, and fixation was carried out at 4°C for 10 minutes.

Thereto was added PBS(-) to make the total volume 1 ml, and centrifugation was carried out at 2,000 rpm for 5 minutes. After the same procedure was carried out once again, the cells were suspended in 1 ml of PBS(-) and stored at 4°C (fixation).

To the fixed cells, 700 µl of perm solution (IC Perm^{TM} Cell Permeabilization buffer, BIOSOURCE International) was added, vortexed, and left at room temperature for 4 minutes. After centrifugation was carried out at 2,500 rpm for 3 minutes, the supernatant was removed except for 40 µl of the perm solution in the tube. Further, centrifugation was carried out at 2,500 rpm for 3 minutes, and the perm solution was removed completely. To the cell pellet, an Alexa-labeled mouse anti-flavivirus monoclonal antibody (clone 6B6C-1) was added, well suspended, and incubated on ice for 30 minutes in the shade. After centrifugation was carried out at 2,500 rpm for 3 minutes, the supernatant was removed. Then, the cells were suspended with 1 ml of PBS(-), and left on ice for 5 minutes (washing).

The same procedure was further carried out twice, and finally, 500 to 1,000 ml of PBS(-) was added to the cell pellet and well suspended. Then, detection of an antigen positive cell was carried out with a flow cytometer.

The results are shown in Fig. 9. The paragloboside saccharide chain inhibited the infection of K562 cells with dengue virus depending on the concentration thereof in the same manner as binding inhibition. Therefore, it was demonstrated that the paragloboside saccharide chain and the related saccharide chain derived from an insect are effective as a potent dengue virus infection inhibitor. Further, it was confirmed that the inhibitory effect of the paragloboside saccharide chain on dengue virus binding does not depend on the bound substrate.

### Industrial Applicability

As described in detail above, by the invention of this application, a dengue virus infection inhibitor capable of effectively preventing the infection with dengue virus is provided.

Although dengue fever is listed in the category IV infectious diseases, an effective therapeutic method for the disease has not been established so far, and there were unclear points regarding the mechanism of infection of a host with dengue virus, which is a causal microorganism.

By the invention of this application, a dengue virus infection inhibitor was attained for the first time. At the same time, it has a high degree of usability for serving as a stepping stone to further expansion such as elucidation of the molecular mechanism of cell response due to infection with dengue virus, development of more potent agent for anti-dengue fever or the like.

## Claims

1. A dengue virus infection inhibitor **characterized by** containing, as the active ingredient, at least a carbohydrate molecule having as an essential constituent an oligosaccharide chain represented by the following formula (I):
Hex¹NAcβ1-3Hex²β1-4Hex¹β1- (I)
(wherein Hex¹ and Hex² represent a hexose).

2. A dengue virus infection inhibitor **characterized by** containing, as the active ingredient, at least a molecule represented by the following formula (II):
(X)ₙ-R (II)
(wherein X represents an oligosaccharide chain represented by the following formula (I):
Hex¹NAcβ1-3Hex²β1-4Hex¹β1- (I)
(wherein Hex¹ and Hex² represent a hexose); R is a substrate selected from the group consisting of a hydrogen atom, a substituent having an S, N, O or P atom, a hydrocarbon group, a lipid, a protein and a synthetic polymer, each of which may have a substituent; n is a number of 1 or greater representing the number of the oligosaccharide chains binding to R).

3. The dengue virus infection inhibitor according to Claim 1 or 2, wherein either a hexose represented by Hex³ or an aminohexose represented by Hex³NAc is beta-1-4 linked to the non-reduced end of the oligosaccharide chain represented by the formula (I).

4. The dengue virus infection inhibitor according to Claim 1 or 2, wherein Hex¹ in the oligosaccharide chain represented by the formula (I) is glucose (Glc), and Hex² is galactose (Gal) or mannose (Man).

5. The dengue virus infection inhibitor according to Claim 3, wherein Hex¹ in the oligosaccharide chain represented by the formula (I) is glucose (Glc), Hex² is galactose (Gal) or mannose (Man), and Hex³ is galactose (Gal) or N-acetyl galactosamine (GalNAc).

6. The dengue virus infection inhibitor according to Claim 3, wherein the oligosaccharide chain represented by the formula (I) is paragloboside represented by
Galβ1-4GlcNAcβ1-3Galβ1-4Glcβ1- (Ia).

7. The dengue virus infection inhibitor according to Claim 3, wherein the oligosaccharide chain represented by the formula (I) is
Galβ1-4GlcNAcβ1-3Manβ1-4Glcβ1- (Ib).

8. The dengue virus infection inhibitor according to Claim 3, wherein the oligosaccharide chain represented by the formula (I) is
GalNAcβ1-4GlcNAcβ1-3Galβ1-4Glcβ1- (Ic).

9. A monoclonal antibody to paragloboside represented by the following formula (Ia):
Galβ1-4GlcNAcβ1-3Galβ1-4Glcβ1- (Ia).

10. A monoclonal antibody to an oligosaccharide chain represented by the following formula (Ib):
Galβ1-4GlcNAcβ1-3Manβ1-4Glcβ1- (Ib).

11. A monoclonal antibody to an oligosaccharide chain represented by the following formula (Ic):
GalNAcβ1-4GIcNAcβ1-3Galβ1-4Glcβ1- (Ic).

12. A dengue virus infection inhibitor **characterized by** containing, as the active ingredient, at least a monoclonal antibody according to any one of Claims 9 to 11.
